# EUROPEAN PATENT APPLICATION

(11) **EP 1 985 629 A1**
(43) Date of publication of application: **29.10.2008**
(21) Application number: 07008511.3
(22) Date of filing: 26.04.2007
(51) Int. Cl.: C07K 14/47, A61K 38/16

(54) **Pro-apoptotic molecules and therapeutic uses thereof**

(71) Applicant: UNIVERSITA DEGLI STUDI DI GENOVA, 16126 Genova (IT); Istituto nazionale per la ricerca sul cancro, 16132 Genova (IT)
(72) Inventor: Parodi, Silvio, 16126 Genova (IT); Ponassi, Raffaella, 16126 Genova (IT); Biasotti, Barbara, 16126 Genova (IT)
(74) Representative: Banfi, Paolo

(57) **Abstract**

The present invention provides peptidomimetic compounds which are able to antagonize the pro-survival effects of members of the Bc1-2 protein family, particularly Bcl-XL, thereby inducing apoptosis in the target cells, particularly in tumour cells presenting altered levels of pro-survival and pro-apoptotic factors. The invention further provides pharmaceutical compositions containing such molecules and their use for the prevention or treatment of cancer.

SEQUENCE LISTING

<110> UNIVERSITA' DEGLI STUDI DI GENOVA; ISTITUTO NAZIONALE PER
LA RICERCA SUL CANCRO

<120> PRO-APOPTOTIC MOLECULES AND THERAPEUTIC USES THEREOF

<130> 1796EUR

<160> 9

<170> PatentIn version 3.3

<210> 1
<211> 38
<212> PRT
<213> Unknown

<220>
<223> BH3-like peptide

<220>
<221> MOD_RES
<222> (9)..(9)
<223> Aib

<220>
<221> MOD_RES
<222> (21)..(21)
<223> Aib

<400> 1

Asp Met Arg Pro Glu Ile Tyr Ile Xaa Gln Glu Leu Arg Arg Ile Gly
1               5                   10                  15

Asp Ala Phe Asn Xaa Tyr Lys Lys Trp Lys Met Arg Arg Asn Pro Phe
            20                  25                  30

Trp Ile Lys Ile Gln Arg
        35

<210> 2
<211> 16
<212> PRT
<213> Unknown

<220>
<223> Antennapoedia

<400> 2

Lys Lys Trp Lys Met Arg Arg Asn Pro Phe Trp Ile Lys Ile Gln Arg
1               5                   10                  15

<210> 3

<211>  24
<212>  PRT
<213>  Unknown

<220>
<223>  BH3-like peptide


<220>
<221>  PEPTIDE
<222>  (1)..(1)
<223>  Cell-internalization sequence

<220>
<221>  MOD_RES
<222>  (10)..(10)
<223>  Aib

<220>
<221>  MOD_RES
<222>  (22)..(22)
<223>  Aib

<220>
<221>  PEPTIDE
<222>  (24)..(24)
<223>  Cell-internalization sequence

<400>  3

Xaa Asp Met Arg Pro Glu Ile Tyr Ile Xaa Gln Glu Leu Arg Arg Ile
1               5                   10                  15


Gly Asp Ala Phe Asn Xaa Tyr Xaa
            20


<210>  4
<211>  16
<212>  PRT
<213>  Homo sapiens

<400>  4

Gly Gln Val Gly Arg Gln Leu Ala Ile Ile Gly Asp Ala Ile Asn Arg
1               5                   10                  15


<210>  5
<211>  22
<212>  PRT
<213>  Homo sapiens

<400>  5

Asp Met Arg Pro Glu Ile Trp Ile Ala Gln Glu Leu Arg Arg Ile Gly
1               5                   10                  15


Asp Glu Phe Asn Ala Tyr
            20

<210> 6
<211> 22
<212> PRT
<213> Unknown

<220>
<223> BH3-like peptide

<400> 6

Asp Met Arg Pro Glu Ile Tyr Ile Ala Gln Glu Leu Arg Arg Ile Gly
1               5                   10                  15

Asp Glu Phe Asn Ala Tyr
            20

<210> 7
<211> 22
<212> PRT
<213> Unknown

<220>
<223> BH-3 like peptide

<220>
<221> MOD_RES
<222> (9)..(9)
<223> Aib

<220>
<221> MOD_RES
<222> (21)..(21)
<223> Aib

<400> 7

Asp Met Arg Pro Glu Ile Tyr Ile Xaa Gln Glu Leu Arg Arg Ile Gly
1               5                   10                  15

Asp Ala Phe Asn Xaa Tyr
            20

<210> 8
<211> 28
<212> DNA
<213> Homo sapiens

<400> 8
acatgcatgc ttcagagcaa ccgggagc                                        28

<210> 9
<211> 26
<212> DNA
<213> Homo sapiens

<400> 9
gaagatctgc ggttgaagcg ttcctg 26

## Description

The present invention provides peptidomimetic compounds which are able to antagonize the pro-survival effects of members of the Bcl-2 protein family, particularly Bcl-XL, thereby inducing apoptosis in the target cells, particularly in tumour cells presenting altered levels of pro-survival and pro-apoptotic factors. The invention further provides pharmaceutical compositions containing such molecules and their use for the prevention or treatment of cancer.

### BACKGROUND OF THE INVENTION

The Bcl-2 family is a large group of apoptosis regulators; through the different interactions among themselves they control (positively and negatively) the release of apoptogenic factors such as cytochrome c, calcium ions and several pro-apoptotic proteins that are required for caspases activation [3, 4]. In mammals Bcl-2 is a protein family that includes almost 30 members: all possess at least one of four conserved motifs known as "Bcl-2 homology" domains (BH1 to BH4). This family includes anti-apoptotic proteins like Bcl-2, Bcl-XL, Bcl-w, Al, Mcl1, BOO and two groups of proteins that promote cell death: Bax subfamily proteins (Bax, Bak, Bok, Bcl-XS) and BH3-only subfamily proteins (Bcl-2 Homology Domain 3 Only) (Bid, Bad, Bik, Blk, Hrk, Bim, Noxa, Puma, Bmf, Spike, Bcl-G, Bnip3, Bnip3L, p193) [5, 6]. Pro-survival members contain the BH1 and BH2 domains; all four BH domains are present in Bcl-XL, the anti-apoptotic protein most similar to Bcl-2. Bax sub-family contains BH1, BH2, BH3 domains; BH3-only proteins have only the short BH3 motif. Pro- and anti-apoptotic family members can heterodimerize and inhibit each other in order to keep a delicate balance. The relative concentrations of the three sub-family members behave like a complex switch of programmed cell death.

Defects in the physiological pathways of apoptosis are involved in several pathologies. During the initial phases of cancer development an altered apoptosis can favour the clonal expansion of initiated / preneoplastic cells. At later stages of an autonomous growth or of a fully malignant cancer, defects in programmed cell death can favour cell survival in anoxic conditions or under the toxic effects of antineoplastic drugs.

In haematological malignancies treatment failures can be correlated with defects in the apoptotic pathway [34, 35]. Bcl-XL over-expression is linked to several cancer pathologies and was also reported to generate a statistically and biologically significant trend of multi-drug resistance phenotypes [9, 10]. Bcl-XL represents an interesting molecular target for anticancer therapy, and agents that can down-regulate its expression might represent a new class of useful weapons for cancer treatment.

### DESCRIPTION OF THE INVENTION

The invention provides peptidomimetic compounds (also referred to as "peptidomimetics", "peptides" or "compounds") endowed with pro-apoptotic activity and having the following amino acid sequence (I) (using the one-letter amino acid code):

Bₙ**X**₁ **X**₂ **X**₃ **X**₄ **X**₅IYIAib**X**₆**X**₇L**X**₈**X**₉IGDAF**X**₁₀Aib**X**₁₁Bₘ (I)

wherein:
B represents a cellular internalization sequence, which is preferably selected from;
   KKWKMRRNQFWIKIQR
   KKWKMRRNPFWIKIQR
   GRKKRRQRRRPPQ (TAT 48-60)
   RKKRROrnRRRPPQ (TAT 48-60 Q53Orn)
(Arg)ₙ, wherein n ranges from 4 to 16
PheLeuPheLeu,
and corresponding retro-inverted sequences (Pescarolo MP et al., FASEB J. 2001 Jan;15(1):31-33), wherein the residues at C- or N-terminus can be derivatized with C10 or C14 fatty acids linked by esteric or amidic bonds; preferably B represents the sequence KKWKMRJRNPFWIKIQR;
n and m are 0 or 1, provided they are not both 1; preferably m is 1 and n is 0
**X**₁ is selected from aspartic acid, glutamic acid, asparagine, glycine, alanine, serine;
**X**₂ is selected from methionine, leucine, lysine, valine, isoleucine, alanine;
**X**₃ is selected from arginine, lysine, serine, glutamine, histidine, proline;
**X**₄ is selected from proline, alanine, serine, glutamine, threonine, valine, histidine;
**X**₅ is selected from glutamic acid, aspartic acid, glutamine, alanine, glycine, lysine;
**X**₆ is selected from glutamine, glutamic acid, histidine, lysine, arginine, alanine, proline;
**X**₇ is selected from glutamic acid, aspartic acid, glutamine, alanine, glycine, lysine;
**X**₈ is selected from arginine, lysine, serine, glutamine, histidine, proline;
**X**₉ is selected from arginine, lysine, serine, glutamine, histidine, proline;
**X**₁₀ is selected from asparagine, aspartic acid, serine, lysine, histidine, threonine, glycine;
**X**₁₁ is selected from tyrosine, phenylalanine, histidine, asparagine, cysteine, leucine, threonine;
Aib is alpha-amino isobutyric acid;
wherein the indicated amino acid residues, which are in either L or D configuration, may carry allylic groups attached to the side chains (OH functions in Serine, Tyrosine, or at C alpha in Glycine) or to amidic nitrogens. In a preferred embodiment:
**X**₁ is selected from aspartic acid, glutamic acid and asparagine;
**X**₂ is selected from methionine, leucine and lysine;
**X**₃ is selected from arginine, lysine and serine;
**X**₄ is selected from proline, alanine and serine;
**X**₅ is selected from glutamic acid, aspartic acid and glutamine;
**X**₆ is selected from glutamine, glutamic acid and histidine;
**X**₇ is selected from glutamic acid, aspartic acid and glutamine;
**X**₈ is selected from arginine, lysine and serine;
**X**₉ is selected from arginine, lysine and serine;
**X**₁₀ is selected from asparagine, aspartic acid and serine;
**X**₁₁ is selected from tyrosine, phenylalanine and histidine;
In a more preferred embodiment:
**X**₁ is aspartic acid;
**X**₂ is methionine;
**X**₃ is arginine;
**X**₄ is proline;
**X**₅ is glutamic acid;
**X**₆ is glutamine;
**X**₇ is glutamic acid;
**X**₈ is arginine;
**X**₉ is arginine;
**X**₁₀ is asparagine;
**X**₁₁ is tyrosine;

In a particularly preferred embodiment, the peptide has the following sequence: BₙDMRPEIYIAibQELRRIGDAFNAibYBₘ wherein Aib, B, n and m are as above defined.

In another particularly preferred embodiment, the peptide has the following sequence:
DMRPEIYIAibQELRRIGDADFNAibYKKWKMRRNPFWIKIQR.

These compounds can be prepared with known methods for peptide synthesis, either in solid phase, in solution or using an automatic synthesizer, A typical procedure may for example involve the following steps; i) synthesis in solid phase of the peptide chain on an insoluble polymeric support, removal of the peptide from the resin by hydrolysis with anhydrous hydrofluoric acid or with trifluoroacetic acid in the presence of suitable scavengers, with concomitant deprotection of the side chains, ii) synthesis in solution of fragments of the peptide chain through successive coupling of N-protected amino acids, suitably activated, with an amino acid or a C-protected peptide chain, recovery of the intermediates, successive selective deprotection of the N and C-terminus of said fragments and coupling thereof until the desired peptide is obtained, followed by deprotection of the side chains.

A detailed description of methods and techniques suitable for peptide synthesis can be found in Stewart and Young (1984) Solid Phase Peptide Synthesis, 2nd ed., Pierce Chemical Co.; Tam et al., J. Am. Chem. Soc., (1983) 105: 6442; Merrifield, The Peptides, Gross and Meinenhofer eds. Academic Press (1979) New York, pp. 1-284; Schroeder et al., "The peptides", vol 1, Academic Press, 1965; Bodanszky et al., "The peptides; Analysis, Synthesis, Biology", 2, Chapter 1, Academic Press, 1980.

The compounds of the invention interact with pro-survival proteins, especially with Bcl-XL, thereby inducing apoptosis in the target cell. Strong pro-apoptotic effects were observed in acute myeloid and acute lymphoblastic leukemia cells from patients. No significant evidence of toxicity were observed in non-pathological peripheral blood and bone marrow cells.

The experimental results obtained with a representative peptide show that the molecule is internalized in the target cell, where it drastically decreases mitochondrial potential and activates caspases 9 and 3, thereby triggering the apoptotic cascade which conduces to cell death. The same results were observed in T cell leukaemia, in myelomonocytic leukaemia and in human Burkitt lymphoma cell lines.

Accordingly, a further aspect of the invention relates to the use of a peptidomimetic molecule as above defined for the preparation of a medicament for the prevention or treatment of diseases that involve uncontrolled regulation of the apoptotic process, including polycitemia, thrombocythemia, idiopathic myelofibrosis, especially proliferative diseases such as cancer. Tumours that can be treated with the peptides herein provided include leukaemias, especially acute myeloid and acute lymphoblastic leukaemia, lymphomas, carcinomas, gliomas, sarcomas and solid tumours in general including drug-resistant tumours. In addition, the peptidomimetic molecules can be used in association with other biologically-active substances, in particular with antiproliferative or cytotoxic drugs, e.g. in form of combined preparations for simultaneous or separate use in anti-cancer therapy.

The invention compounds can be combined with pharmaceutically acceptable excipients, such as diluents, carriers, stabilizers, absorption promoters, dispersing agents, emulsifiers, wetting agents, preservatives and/or buffers. The pharmaceutical compositions may be in the form of solid or liquid preparations, such as solutions, suspensions, syrups, suppositories, injectables, capsules, tablets or powders. Aqueous solutions and injectables are preferred dosage forms.

The compositions can be administered intravenously, subcutaneously, intraperitoneally, intradermally, intramuscularly, orally, rectally or intranasally, the parenteral administration route being preferred.

The daily dosage of a peptidomimetic according to the invention may vary depending on the severity and progression of the disease to be treated, on the pharmaceutical form and administration route, on the number of treatments, on the characteristics of the patient (e.g. concomitance of other diseases), but in general an amount between 5 and 25 mg/Kg one or more times daily could be acceptable.

### Description of the figures

**Figure 1**
   Fluorescence Anisotropy Assay. Average anisotropy values: (open square) binding assay for increasing concentrations of Bcl-XL at a fixed concentration of fluorescent Bak-BH3 peptide. Competition assays: (down filled triangle) the above binding assay was competed with a fixed concentration of cold Bim-BH3; (open diamond) a fixed concentration of a modified peptide Bim-BH3Y; (up filled triangle) a fixed concentration of the modified peptide 072RB. The error bars represent standard deviations observed from triplicate experiments.
**Figure 2**
   072RB: cell growth effects. Panel A: Cell growth of U937 cell line treated for four days every 24 h at a 1 µM 072RB concentration and every 48 h at 3, 5, 10 and 30 µM 072RB concentrations. U937 controls at day 4, cell number: 350,000 ±50,000. Number of cells seeded at day 0: 20,000. Arrows indicate treatment time. Panel B: 072RB affects cell growth of leukaemic monocyte cell line U937, the T cell leukaemia cell line Jurkat, the Burkitt lymphoma cell line Namalwa. Cells are treated twice every 48 h at a 10 µM concentration and followed for four days. Number of cells in untreated control cultures at day four: Namalwa; 330,000 ± 70,000; Jurkat: 300,000 ± 65,000; U937: 350,000 ± 50,000. Number of cells seeded at day 0: 20,000. All cells were counted in a Burker chamber and values are expressed as percentage of the untreated control cell number. Only living cells identified by trypan blue exclusion staining were counted. The error bars represent standard deviations observed from triplicate experiments.
**Figure 3**
   072RB induces apoptosis of tumour cell lines. Panel A 072RB induces apoptosis of U937 cells. Flow cytometry measurements of DNA content (evaluated by propidium iodide (PI) fluorescence intensity) and physical cell side-scattering (evaluated by SSC) in U937 cells treated with Int and 072RB at 10 µM, twice each 48h. Bivariate PI/SSC dot plots are reported in the first and third row and the respective DNA content univariate distributions underneath (projection of the dot plots on the x-axis). Three main cell populations can be observed in the bivariate PI/SSC plots, one with low SSC and normal DNA content (that accounts for "live" cells in culture), another with high SSC and normal DNA content (cells undergoing "early stage of apoptosis") and another one with lower PI fluorescence (cells at "late stage of apoptosis" and debris). Panel B 072RB induces apoptosis of tumour cell lines. Fraction of alive, early- and late-apoptotic cells in tumour cell line cultures treated for four days with 5 and 10 µM 072RB or the vector Int alone at 10 µM. At least three independent experiments were performed, and mean values (± standard errors) are reported.
**Figure 4**
   072RB induces mitochondrial permeability transition (MPT) of tumour cell lines. Panel A: Flow cytometry DiOC6 fluorescence histograms of U937 cells treated for 4 days with 5 and 10 µM 072RB respectively. Decreased fluorescence of the marker indicates reduction of the mitochondrial inner membrane potential, i.e, MPT (a representative experiment). The percentage of cells undergoing MPT is reported. Panel B: Percentage of cells with reduced DiOC6 fluorescence in cultures of U937 and Jurkat cell lines treated with 072RB for 4 days. Results are expressed as the mean (± SD) from at least two independent experiments.

### EXPERIMENTAL PROCEDURES

### MATERIALS AND METHODS

### Recombinant Bcl-XL (ΔTM) Protein expression and purification

A DNA fragment corresponding to the coding sequence for human Bel-XL (Accession No. Z23115.1), with the C-terminus truncated, was amplified by PCR using the following oligonucleotides: forward 5'-ACATGCATGCTTCAGA,GCAACCGGGAGC- 3'(SphI site)
and reverse 5'-GAAGATCTGCGGTTGAAGCGTTCCTG- 3' (BgIII site).

The second codon of Bcl-XL was altered to create a SphI site. The amplified product and the His6 vector pQE-70 were digested with SphI and BglII, followed by ligation and transformation into JM109 strain Escherichia coli in Luria-Bertani medium supplemented with ampicillin (100 µg/ml) and Chloramphenicol (25 µg/ml). Colonies were screened by restriction enzyme digestion of plasmid DNA. The DNA correct sequence was confirmed through sequencing analysis. The PCR product was inserted in the PCRII vector (TA cloning system; In Vitrogen, San Diego, CA) and then cloned in the bacterial expression vector pET-14b(+)-His- TAG (Novagen; Madison, WI) between the Nde I and BamH I sites. Vectors were sequenced by an ALF DNA sequencer (LKB-Pharmacia Biotech) using both vectors and internal primers. Escherichia coli BL21 (pLys) were transformed with purified plasmid and recombinant protein expression was induced for up to 15 h by adding 100 mM IPTG to exponentially growing bacteria at room temperature. Bacteria were collected by centrifugation and resuspended in 5 mM imidazole, 0.5 M NaCl, 20 mM Tris-HCl (pH 7.9); samples were sonicated on ice. Following centrifugation at 12,000 g for 20 min, bacterial lysates were then applied to a charged and equilibrated Chelating Sepharose (Pharmacia Biotech) chromatography column. The column was then washed with 10 volumes of 5 mM imidazole, 0.5 M NaCl, 20 mM Tris-HCL (pH 7.9) (binding buffer), and 6 volumes of 50 mM imidazole, 0,5 M NaCl, 20 mM Tris-HCl (pH 7.9) (wash buffer). The bound protein was eluted with 6 volumes of 1 M imidazole, 0.5 M NaCl, 20 mM Tris-HCl (pH 7.9 elution buffer). Fractions were collected and purity of recombinant His₆-Bcl-XL (ΔTM) protein preparation was determined by SDS-polyacrylamide gel electrophoresis. The eluted recombinant protein (26,000 Mᵣ) was found predominantly monomeric by centrifugal filtration on a 30,000 NMWL Millipore filter unit and capable of immunoreaction with Bel-XL antibodies.

### Peptides Synthesis and Modification

The sequences of the peptides used in this work are Bak-BH3D84A (for short BakF-BH3):
72GQVGRQLAIIGDAINR87;
Bim-BH3: 83DMRPEIWIAQELRRIGDEFNAY104;
Bim-BH3Y: 83DMRPEIYIAQELRRIGDEFNAY104;
Bim-BH3YA2Aib: 83DMRPEIYIAibQELRRIGDAFNAibY104;
Int Q50P: 58KKWKMRRNPFWIKIQR43;
072RB:
   DMRPEIYIAibQELRRIGDAFNAibYKKWKMRRNPFWIKIQR.
All peptides were chemically synthesized by Fmoc (9-fluorenylmethoxycarbonyl) [31] solid phase synthesis on a Champion PAG PEL Resin (Advanced Biotech) with substitution level 0.37 mmol/g. After deprotection of the Fmoc group attached to the solid support with 20% piperidine in DMF at 35°C (20 minutes treatment time), the resin was treated with a coupling reaction mixture containing 4 eq of the appropriate Fmoc amino acid (Advanced Biotech), 3.8 eq of o-(7- azabenzotriazol- 1- yl)- 1, 1, 3, 3 tetramethyluroniumhexafluorophosphate (HATU) (Advanced Biotech), 4 eq of N,N-di-isopropylethyl amine (Fluka Chemie AG, Buchs, Switzeland) and 6 eq of sim-collidine (Fluka) at a 0.2 M amino acid final concentration in anhydrous N-methylpirrolidone (NMP Merck) (for 40 minutes to 2 hours at 35°C). After synthesis the peptide was cleaved from the resin through the trifluoroacetic acid (TFA Pierce) procedure and precipitated in ice cold diethyl ether by standard methods. The fluorescein-labelled peptides were synthesized adding a fluorescein molecule at the N terminal, treating the deprotected resin with 1,5 eq of 5,6 carboxy fluorescein N-hydroxysuccinimide ester (Fluka) and 3 eq of DIEA (Fluka) in NMP for 16 hr at 25°C.

The crude product obtained was purified with a Shimadzu LC-8A preparative HPLC apparatus equipped with a Waters C18 Bondapack column (19 mm-300 mm). The diode array detector, during analysis, was set at 220 nm and a gradient from 100% of water (0.1% TFA) to 100% of acetonitrile (0.1 % TFA) was applied. The molecular mass and purity of the obtained peptides were determined and confirmed by HPLC-MS (see below).

### Mass spectrometry: analysis and characterization

All samples, including the chemically synthesized compounds and their extracts, were analyzed by directly coupling HPLC to a mass spectrometer. MS analysis of peaks arising from HPLC (an Agilent 1100 instrument equipped with a Waters Symmetry 300TM C18 1x150mm column) was performed by directly introducing the mobile phase at a flow rate of 30 µl/min in the mass spectrometer orthogonal electro-spray ion source. The peptides separation was performed in a gradient starting at 100% solvent A and linearly increasing to 100% solvent B in 35 min; then isocratic conditions at 100% solvent B were applied from 35 to 40 min. Solvent A was water with 0,1% TFA and B was acetonitrile with 0.08% TFA. The diode array detector wavelength was set at 220 nm.

Mass spectra of all samples analyzed were acquired in the positive ion mode in a mass range including the expected m/z ratios for the triple and/or quadruple charged moieties, using an Ion Trap Agilent 1100 Series LC/MSD Trap equipped with an orthogonal electrospray ion source.

The mass spectrum of the purified peptides water; acetonitrile 1:1 solutions showed a deconvoluted neutrally charged molecular ion at m/z 2697 for Bim-BH3, m/z 2667 for Bim- BH3YA2Aib and m/z 4868 for 072RB consistent with the expected peptides molecular weights. The same results were obtained on the extracted peptides; in that case 8 µl of the extraction solution were directly injected in the HPLC system.

### Molecular modelling

Since no structure of the human Bcl-XL:Bim heteroduplex is currently available, the model was built on the corresponding crystallographic mouse complex available in the Protein Data Bank (1PQ1 code). The structure was read into Sybyl 7.0 and carefully checked for chemically consistent atom and bond type assignment. Hydrogen atoms, not present in the PDB files, were added using Biopolymer and Build/Edit menu tools and minimized at 0.5 kcal (mol Å)⁻¹ for 1500 cycles to avoid steric clashes and repulsive acid/acid interactions. Only waters placed in a 4 Å range between the protein and peptide were retained and analyzed. The sequence alignment between the mouse Bel-XL (complexed with the Bim peptide; 1PQ1 code) and the human Bcl-XL, (native form; 1BXL code) was performed using ClastlX [32] and GeneDoc [33].

The Bim structure present in the mouse complex consisted of 33 aminoacids; only 22 residues were retained, according with the size of the peptide used in the experimental measurements. Four residues were mutated, to transform the mouse Bim into the corresponding human Bim. Site-directed mutagenesis in silico was carried out using the molecular modelling program Sybyl version 7.0 [34]. According with the experimental procedure, Trp89 on the Bim peptide was then mutated into Tyr89. All Molecular Dynamics simulations were performed using the Tripos force field implemented in Sybyl.

The program HINT (Hydropathic INTeractions) [35], was used to evaluate the strength of the interaction between Bcl-XL and Bim peptides before and after the Trp89 to Tyr mutation. The calculations were performed as previously reported [36, 37] using a HINT version (3.10Sβ) with local modifications to the commercial version [34]. Partition calculations were performed with the dictionary method for both the protein and the peptide. Neutral solvent conditions were applied.

Since no water molecule was considered especially significant for the current analysis, the protein-water contribution was not added to the protein-protein HINT score.

### Circular Dichroism (CD)

Circular dichroism (CD) spectra in the far-UV region (190-260 nm) were collected on a Jasco J-715 spectropolarimeter using a 0.1 cm path-length cuvette. Temperature was kept at 25°C by a Peltier device. Peptide concentration was determined from the absorption of the aromatic residues at 280 nm. Extinction coefficients of the peptides, calculated from the values for model compounds¹, are as follows: 2,560 M⁻¹, cm⁻¹ for Bim-BH3Y and Bim-BH3 YA2Aib and 13,940 M⁻¹, cm⁻¹ for 072RB.

Peptides solutions: Bim-BH3Y and Bim-BH3YA2Aib were suspended in water and potassium hydroxide was added up to complete solubilization. 072RB was dissolved in water. Concentration of stock solutions was about 0.1 mg/ml. For circular dichroism measurements, stock solutions were diluted in 10 mM potassium phosphate buffer, pH 7.

### Fluorescence Anisotropy

Fluorescence Anisotropy was monitored with a Polarion Instrument (Tecan, Austria): excitation wavelength = 485 nm, emission wavelength = 535 nm. Recombinant Bcl-XL, fluorescein-labeled Bak-BH3 peptide, Bim-BH3 and mutant peptides were diluted in assay buffer: 25mM NaHCO₃, 120 mM KCl, 1mM KH₂PO₄, 10 mM MgCl₂ and 20 mM Hepes, pH 7.4. Anisotropy values were studied at a fixed concentration of fluoresceinated Bak BH3 molecule (2E-8 M), a fixed concentration of peptide inhibitors (2E-7 M) and increasing concentrations of Bel-XL (from 2E-8 M to 1E-6 M). Experiments were performed in 96-wells black non-binding plates (Corning), the experimental volume was always 200 µl / well. Reagents were mixed at room temperature and maintained at a constant temperature (30°C) during the measurements. Anisotropy measurements were performed at different times of incubation to assess the stability of the reactions. Results refer to measurements after 45 min of incubation, sufficient to reach an equilibrium state.

### Data analysis

For simple binding experiments we evaluated the K_{d} using a nonlinear regression analysis [NLIN SAS] to fit the Lundblad equation [38]. To analyze results of competitive binding inhibition assay we performed a mixed parametric/non parametric bootstrap analysis [39].

We calculated the expected Anisotropy value solving a third degree equation obtained extending the simple binding equation to a competitive binding inhibition assay. Comparing experimental data with expected anisotropy, we detect the value of K_{d} minimizing the Sum of Squared Differences for the bootstrap sample.

Point estimates of each equilibrium constant were evaluated using meta-analytical tools weighting each experimental result for its estimated variance. Standard error values reported in Table 2 are computed as squared root of pooled variance among different assays.

### Peptides stability

The Bim-BH3 peptides were extracted from medium (RPMI 1640), supplemented with 10% FBS and from 100% FBS. Compounds were added to the medium solution of serum 10% or 100% FBS at the final concentration of 32 µM. Several extraction mixtures were attempted: MeOH; MeOH with 0,1% trifluoroacetic acid (TFA); (H2O: MeOH) 1:1, 1:4, 4;1. A perchloric acid (PCA) protein precipitation protocol was also tried. For Bim-BH3 and mutants Bim-BH3YA2Aib peptides a complete and specific extraction was obtained using methanol at the final concentration of 50% v/v, while for 072RB molecule the best result was achieved using methanol added with 0.1% TFA. In both cases ice-cold methanol was added to the peptide solution. The mixture was centrifuged and the resulting supernatant was then added with ice-cold methanol (alone or containing 0,1% TFA) at a 1:1 final ratio and this procedure was repeated twice. Before extraction peptides were incubated in 10% FBS or 100% FBS at 37°C and, at different incubation times (0', 6, 24 and 48 hours), a sample of 50 µl was collected to be analyzed by HPLC-MS.

### Cell Culture

The leukemic myelomonocytic cell line U937, the T cell leukaemia cell line Jurkat, the Burkitt lymphoma cell line Namalwa were grown in RPMI 1640 medium supplemented with 10% FBS, 1% sodium pyruvate, 1% non essential aminoacids, 1% penicillin/streptomycin. 2.5·10⁴ cells were seeded on 48 wells plates (Corning Costar). The treatment was performed on triplicate wells. Drug concentration ranged from 1 µM up to 30 µM, four treatments every 24 h or two treatments every 48 h were performed. Human peripheral blood lymphocytes obtained from healthy volunteers by venipuncture, were isolated by lymphocyte separation medium (Ficoll) density gradient centrifugation. Mononuclear cells from the interphase were washed twice with PBS and resuspended in RPMI 1640 medium containing 10% FCS, glutamine and antibiotics. Phytohemagglutinin (PHA) was added at 1.5 µg/ml final concentration.

For experiments with primary cells of acute lymphoblastic leukaemia (ALL), Chronic Acute Myeloid Leukaemia (AML), fresh samples of bone marrow aspirates were from patients who had at least 80% blasts among their mononuclear cells. Normal hematopoietic cells (NBM) were obtained from healthy volunteers. Mononuclear cells were isolated from the samples by Ficoll density-gradient centrifugation. Primary cells were cultured as above. The collection of human specimens was performed after informed consent of the patient during diagnostic procedure. Viability was determined by Facs analysis: PI emission fluorescence.

### Flow cytometry of cells incubated with 072RB peptide and microscopy analysis of 072RB peptide internalisation

Cell lines (10⁶ cells / well, in a 24 wells plate) were incubated with different concentrations of 072RB fluoresceinated peptide (1, 10 µM) for 30 min, 60 min, 120 min, 180 min, 18 h, 24 h, 48 h, 72 h, in complete RPMI medium at 37°C. Cells were extensively analysed on a flow cytometer (FACSort, Becton Dickinson). Parallel samples were incubated with the same amounts of Int or with 072RB fluoresceinated peptides to assess the degree of internalisation. At least 10⁵ events were analysed for each sample, using the Cell Quest computer program (Becton Dickinson), Data are expressed as Log green fluorescence intensity (arbitrary units) vs number of cells.

The same samples were also analysed under the inverted microscope IX71 (Olympus Biosystem) equipped with a charged coupled device camera and the CELLR 2.0 program. Images were taken and analysed with analySIS Five soft imaging system to quantify the relative amount of FITC-peptide present inside the cells compared to the extracellular compartment, results were plotted as mean fluorescence intensity (MFI) expressed in arbitrary units. MFI of cells analysed on flow-cytometer and images taken with CCD camera are expressed in different arbitrary units.

### Confocal microscopy of 072RB intracellular localization

Cell lines (10⁶ cells / well, in a 24 wells plate) were incubated with different concentrations of 072RB fluoresceinated peptide (1, 10 µM) for 30 min, 60 min, 120 min, 180 min, 18 h, 24 h, 48 h and 72 h, in complete RPMI medium + 10% serum at 37°C. During the last 30 min cells were incubated with either Lysotracker red DND-99 (LTR, 50 nM) or Mitotracker Red 580 (MT, 100 nM) (Molecular Probes, Poortgebouw, Leiden, The Netherlands), Samples were seeded on a micro-slide; a cover slip was sealed on the slide adding minute amounts of nail polish at the four sides; cells were analysed with an IX81 microscope equipped with a 488 nm argon laser and a He-Neon 523 nm laser (FV500, Olympus BioSystem). 10-15 fields were analysed with 40x oil/1.35 NA or 60x oil/1.40 NA objectives and images acquired using the Flowview 4.2b computer program. The relative amount of fluoresceinated-peptide present in different intracellular compartments was compared with the amount present in the extra-cellular compartment. Results were plotted as a mean fluorescence intensity (MFI) expressed in arbitrary units. MFI of cells determined by confocal microscopy or flow cytometry or images taken with CCD camera are expressed in different arbitrary units.

### DNA flow cytometry for apoptosis and cell proliferation evaluation

Cells were harvested and incubated with 30 µg/ml PI (Sigma Chemical Co.) and 0.5 mg/ml RNase for 30 min at room temperature and in the dark. Flow cytometric measurements of Forward Scatter (FSC) and Side Scatter (SSC) of light emitted from the excitation laser, and of PI emission fluorescence, were performed on a FACSCalibur (Becton Dickinson) flow cytometer. At least 10,000 events were acquired on a linear scale from each sample. Frequency distributions of DNA content and SSC were analysed for the evaluation of apoptosis at different apoptotic stages and cell cycle phases, as previously described.

### Propidium iodide (PI) uptake test

PI uptake tests were performed to assess cell viability. Cells suspended in culture medium were incubated for 5 min with 3 µg/ml PI and analysed by flow cytometry. Cells that did not take up the fluorochrome (*i.e.* negative fluorescence) were considered as viable, whereas fluorescent cells, i.e. cells that took up the dye due to a damaged plasma membrane, were considered as dead (late apoptotic + necrotic cells). At least 10,000 events were acquired on a logarithmic scale from each sample.

### Evaluation of caspases 9 and 3 activation and apoptosis.

The activation of caspase-9 (mitochondrial apoptosis) and caspase-3 (activated downstream of caspase-9), was evaluated in cell lines (10⁶ cells/well in a 24 wells plate) incubated with different concentrations of 072RB (1, 10 µM for 24h, 48h and 72h, in complete RPMI medium + 10% FCS at 37°C, using the caspase-9 and caspase-3 Detection Kit (Calbiochem, Darmstadt, Germany). The assay utilizes LEHD-FMK or DEVD-FMK conjugated to fluorescein-isothiocyanate (FITC) as caspase-9 or caspase-3 in situ marker. FITC-LEHD-FMK and FITC-DEVD-FMK are cell permeable, non toxic, and irreversibly bind only to activated caspase-9 or -3 in apoptotic cells. Samples were run on a flow cytometer (FACSort, Becton Dickinson) equipped with an argon ion laser exiting FITC at 488nm, At least 10⁵ events were analysed for each sample using the CellQuest computer program. Data are expressed as Log green fluorescence intensity (a.u.) vs cell number. Calibration was assessed with CALIBRITE particles (Becton Dickinson) using the AutoCOMP computer program.

Morphologic analysis of apoptosis was performed on cells incubated with 072RB or Int peptide for 48-72 h, fixed in 70% ethanol/PBS solution (5min at 4°C), permeabilized with 1% NP-40 detergent, for 5min at 4°C. After extensive washes, samples were incubated with PI (50µg/ml in PBS) and 100 units/ml Rnase type A and 10 mM EDTA (to inactivate endogenous nucleases) for 15 min at room temperature. After three washes, cells were put on a micro-slide, covered with a coverslip and analysed with an IX81 microscope equipped with He-Neon 523 nm laser, suitable for PI fluorescence excitation (FV500, Olympus BioSystem). 10-15 fields were analysed with 40x oil/1.35NA or 60x oil/1.40NA objectives and images acquired with the Flowview 4.2b computer program.

### Detection of Mitochondrial Permeability Transition

Induction of mitochondrial permeability transition (MPT) was evaluated by the decrease in mitochondrial inner membrane potential as previously described (Zamzami J Exp Med 1995). Cells were incubated with 40 nM 3,3'-dihexyloxacarbocyanine iodide (DiOC₆) for 15 min at 37°C and analysed by a FACSCalibur equipped with a 488 nm laser suitable for DiOC₆ excitation and cut-on optical filters suitable for DiOC₆ emission-fluorescence (552 nm). At least 10,000 events were acquired per sample on a logarithmic scale. The suppression of trans-membrane potential causes a shift of DiOC₆ fluorescence to lower levels.

### RESULTS

### Polarization assay

Fluorescence Anisotropy (FA) is a quantitative technique to analyze the interaction of a smaller fluorescent molecule with a larger non fluorescent molecule. We used this biochemical approach to identify Bcl-XL inhibitors [25, 26]. To obtain a reliable quantitative outcome this technique requires a careful methodological approach and an appropriate mathematical/statistical elaboration of the experimental data (see Methods).

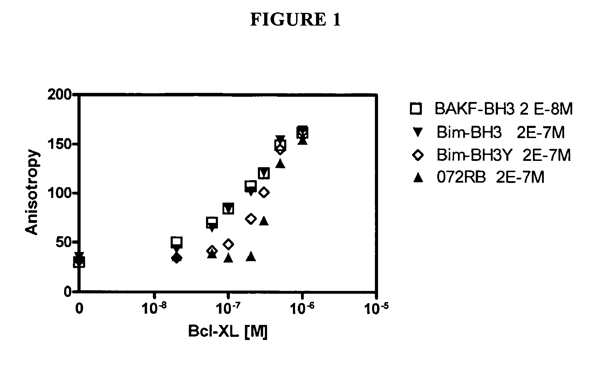

We performed (Figure 1) an initial binding assay measuring the interaction between Bcl-XL and a fluoresceinated Bak-BH3 peptide (BakF-BH3) bearing an amino acid substitution. Within the BH3 motif this substitution can be indicated as D84A. A K_{d} had already been reported for this protein-protein interaction (K_{d} =1.4 E-7M) [27], As shown in Table 1 we found a very similar value.

To analyze the interaction between Bcl-XL and Bim-BH3-derived peptides we set up a competition binding assay: at a fixed concentration of BakF-BH3 peptide (2E-8 M) and a fixed concentration of a Bim-BH3-derived peptide (2E-7 M), we measured anisotropy values for increasing Bcl-XL concentrations (from 2E-8 M to IE-6 M). In the presence of a Bim-BH3 concentration 10 times greater than the one of BakF-BH3 we did not observe a relevant decrease of anisotropy values at Bel-XL concentrations roughly between 1E-7 M and 3E-7 M (the most sensitive range). On the contrary, for the same respective molar ratios, an evident decrease of anisotropy values was observed for the Bim-BH3 mutant peptide in which aromatic amino acid Trp89 was substituted with Tyr. This peptide showed a higher affinity for the Bcl-XL hydrophobic cleft, as estimated by data analysis and best-fitting computation of K_{d} values (Table 1). Moreover, we further increased affinity using as a competitor a Bim-BH3 mutant peptide in which the aromatic amino acid Trp 89 was substituted with Tyr, Glu 100 with Ala and two Ala 91, 103 were substituted with two α-amino isobutyric acid (Aib). Aib was introduced following the suggestions of literature reports showing that an alpha helical conformation is favoured by the presence of Aib aminoacids [28, 29]. A further increase of affinity was obtained linking the molecule to an internalisation tag, a modified Q50P 16 aa Antennapedia III^{rd} α-helix (Figure 1 and Table 1). The above substitution had been reported to induce a preferential cytoplasmic, rather than nuclear, location of the internalised molecules [19, 30].

**Table 1: Dissociation equilibrium constants between Bel-XL and five different peptides.**

| **Peptide** | **Weighted mean Kd** | **Pooled SE** |
|---|---|---|
| BakF-BH3 (D84A) | 1,384E-07M | 1.563E-08 |
| Bim-BH3 | 3.342E-07M | 3.584E-08 |
| Bim-BH3 Y | 2.916E-08M | 5.88E-09 |
| Bim-BH3YA2Aib | 1.196E-08M | 4,252E-09 |
| 072RB | 4.304E-09M | 1.030E-09 |

### Molecular Modeling

The mouse complex (for which XR data are available [18]) involves the formation of several H-bonds and hydrophobic interactions between Bcl-XL and Bim-BH3: Q125-R85, Q111-R89, E129-Q92, E129-R95, N136-D99, Y101-E100, Y195-N102 [18]. No significant contact was lost after the modeling of the human complex from the mouse crystallographic structure, except Q111-R89, since R89 was mutated into W89. The noteworthy sequence homology and the hypothetical interface similarity led us to consider the derived complex a reliable model of the human Bcl-XL and Bim-BH3 association. The mutated human complex was energetically optimized with a MD procedure. The HINT force field was used to quantitatively estimate the interaction strength of the human complex before and after the W89Y mutation. The comparison of the wild type and mutant interface clearly shows the formation of two additional H-bonds, between Tyx89 on Bim-BH3Y peptide and Arg103 and Gln111 on Bcl-XL. The HINT score difference between the wild type and the mutant complex (364 HINT score units) could be reasonably compared with the experimental ΔG° difference (0.8 kcal/mol).

### Circular Dichroism

Structural data indicate that BH3 domains when bound to their target proteins adopt an α-helical structure [27, 31] which can be lost in isolated peptides. The relative content of secondary structure elements was calculated with the deconvolution program CDSSTR [31] (Table 2). The substitution of Ala with Aib should promote α-helix stabilization [28, 29]; as a matter of fact the relative content of helices shows a five-fold increase in Bim-BH3YA2Aib with respect to Bim-BH3Y (from 0.03 to 0.15 α-helix percent). 072RB has the highest helical content in the set examined here (0.30 α-helix percent). The increase in helix content with respect to Bim-BH3YA2Aib could certainly be due to additional contribution to helix stabilization of the internalization peptide (at the carboxi-terminal of our molecule) and it may favor a more potent pro-apoptotic activity [33].

**Table 2: CD results: Relative content of secondary structure elements of Bim-BH3 derived peptides.**

| **Peptide** | **α-Helix** | **β-Strands** | **β-Turns** | **Random** | **Total** |
|---|---|---|---|---|---|
| Bim BH3 Y | 0.03 | 0.22 | 0.14 | 0.59 | 0.98 |
| Bim BH3 YA2Aib | 0.15 | 0.14 | 0.15 | 0.56 | 1.00 |
| 072RB | 0.30 | 0.12 | 0.15 | 0.42 | 0.99 |

### Peptides stability

The half-life studies of Bim-BH3 peptides allowed, at the considered incubation times, the relative quantification of the whole peptide measuring UV and Total Ion Current (TIC) chromatograms peak areas. The half-life in medium with 10% FBS, calculated by linear regression, is for Bim-BH3Y 49.5 h and for Bim-BH3YA2Aib 99,0 h, showing a clear stabilizing effect derived from the incorporation in the peptide sequence of two residues of the unnatural amino acid Aib. The 072RB half life is 36.5 h in medium with 10% FBS and 17.8 h in 100% FBS; the internalization sequence Int was perhaps digested more efficiently by peptidases, but we are still in the presence of a quite stable molecule.

### Internalization of 072RB

To determine whether 072RB could enter cells, we performed a series of experiments incubating three different tumor cell lines: U937, Namalwa and Jurkat with fluoresceinated 072RB (1, 10 µM) for different periods of time (0, 60, 120, 180 min). 072RB, Bim-BH3YA2Aib (as a negative control) and Int (as a positive control) fluoresceinated peptides were then analyzed on a Facs and a microscope equipped with a CCD camera. Mean fluorescence intensity (MFI, in arbitrary units) compared with fluorescence in the extra-cellular medium (MFI: 160-190), was considered a relative quantification of the amount of peptide entered inside the cells. The amount of peptide recovered inside the cells was going to be relevant after 180 min in each of the three cell lines. The amount of fluoresceinated 072RB entering the cells was comparable to that of fluoresceinated Int (data not shown). On the contrary, fluoresceinated BimBH3YA2Aib (lacking the Int internalization sequence) entered the cells at a much lower level, at 180 min. These findings indicate that Int sequence is necessary for an efficient internalization. 072RB was able to penetrate with similar kinetics both B and T cell cancer lines; in U937 cells a plateau was observed at 18-24 h (MFI: 1700 ± 150, n=3).

Furthermore, we could quantify the relative amount of 072RB fluoresceinated peptide entered in the cells analyzing the MFI of organelles, cytoplasm or extracellular milieu of Namalwa cells after 24 hours of incubation. Indeed, MFI inside the organelles (range MFI 460-1200) was stronger than that observed free into the cytoplasm (range MFI 150-220) and outside the cells (range MFI: 160-190).

### Intracellular localization of internalized 072RB

To determine whether 072RB co-localizes inside the cells into mitochondria, tumor cell lines have been incubated with fluoresceinated 072RB for 3 h rather than 24 h. Mitotracker red, a niltochondrion-selcetive probe, was added during the last 30 min of incubation. Fluoresceinated 072RB mainly co-localizes, with mitotracker red in mitochondria. It is worthwhile to note that this peptide did not stain lysosomes, as shown by lack of co-localization with cellular organelles labeled with Lysotracker Red DND-99, a fluoresceinated probe specific for lysosomes. In conclusion, 072RB co-localizes with mitochondria, but not with lysosomes.

A quantification of the amount of 072RB inside the cells at 3 h could also be performed, and MFI determined in different cell compartments. This analysis revealed that the amount of fluoresceinated 072RB inside mitochondria was approximately 15 fold higher than the one present into the cytoplasm; MFI 1500 ± 150 a.u. in mitochondria vs 100 ± 40 a.u. in the cytoplasm; in the extra cellular milieu levels of 70 ± 20 a.u. were observed.

Arbitrary fluorescence units of different experiments (for instance observations at 24 and 3 h) can be compared only in relative, but not absolute, terms.

### Cell death and MPT (mitochondrial permeability transition)

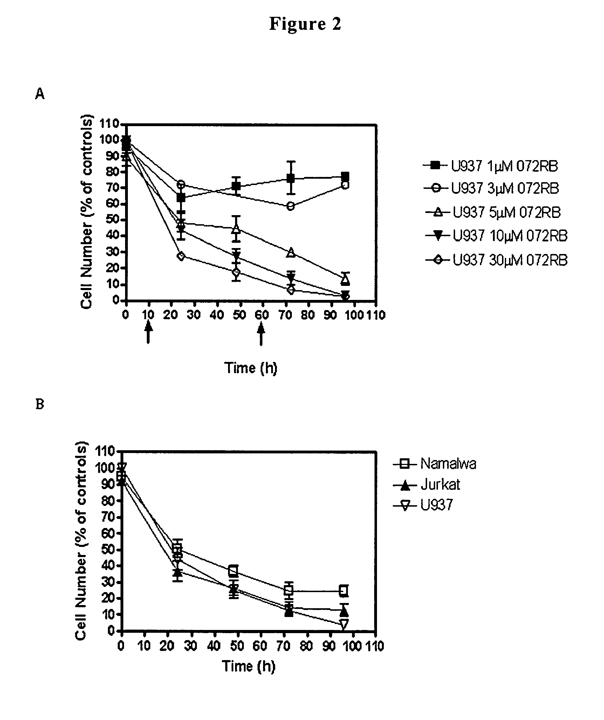

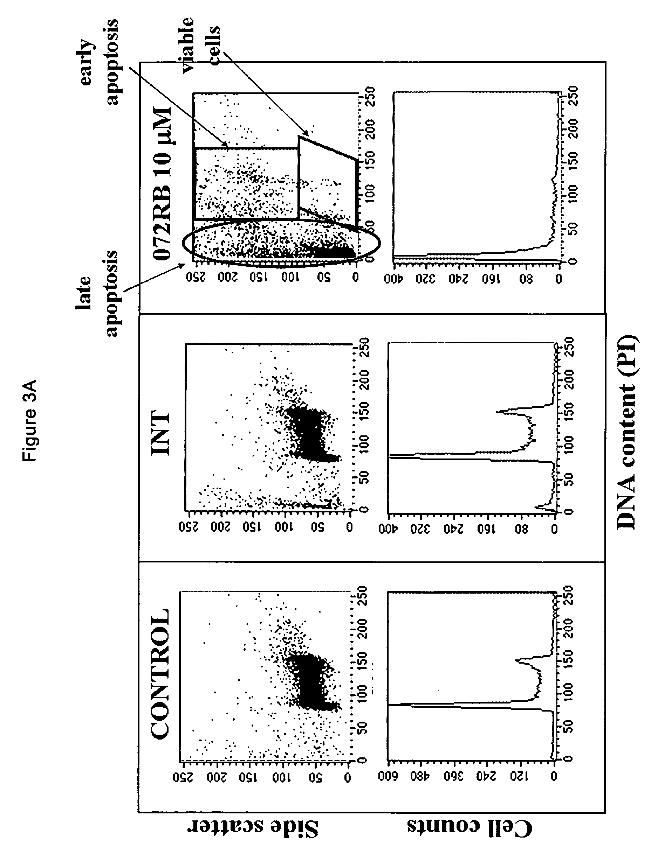

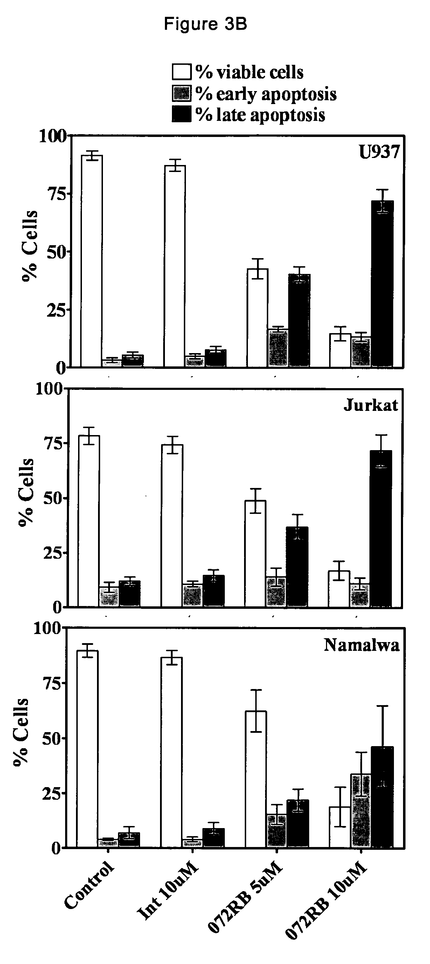

U937 cell counts of 072RB treated cultures, with concentrations ranging from 1 µM up to 30 µM, indicate a dose-dependent inhibition of cell growth (Figure 2A). Based on the results reported above we selected treatment concentrations of 5 µM and 10 µM for subsequent experiments. Considering the measured half-life of 072RB in tissue culture medium containing 10% serum at 37°C, 072RB or the Int sequence were added to cultures twice (every 48 h). A progressive decrease of cell growth compared to control cultures can be observed during time for the three cell lines treated with 10 µM 072RB, as reported in Figure 2B. To evaluate whether the decreased cell growth was due to cell cycle arrest, cell death, or both, flow cytometry assays to assess cell viability and cell cycle behavior were performed. PI (Propidium Iodide) uptake tests, used to assess cell viability, showed dose-dependent cell death of tumour cell lines treated with 072RB for 4 days, but not with the internalization sequence Int alone (data not shown). Flow cytometry measurements of DNA content and side scatter confirmed that the main cause of cell growth inhibition after 2 or 4 days of 072RB treatment at 5, 10 or 30 µM respectively, was cell death rather than cell cycle perturbation, as shown in Figure 3A for U937 cells. Three main cell populations can be observed in bivariate plots of side scatter (SSC) vs. DNA content (PI fluorescence), one with low SSC and normal DNA content (that accounts for cells "alive" in culture), another with high SSC and normal DNA content (cells undergoing "an early stage of apoptosis ") and another one with lower PI fluorescence (cells at "a late stage of apoptosis" and debris). The respective DNA histograms (projection of the dot plots on the x-axis) are reported for each plot. Data after 4 days of 072RB treatment at 10 µM respectively on all three tumour cell lines are reported in Figure 3B, displaying the fraction of cells distributed in the three above sub-populations. Significant cell death can be observed, with an increasing trend going from 5 to 10 µM 072RB. Int 10 µM was substantially inactive in all three cancer lines.

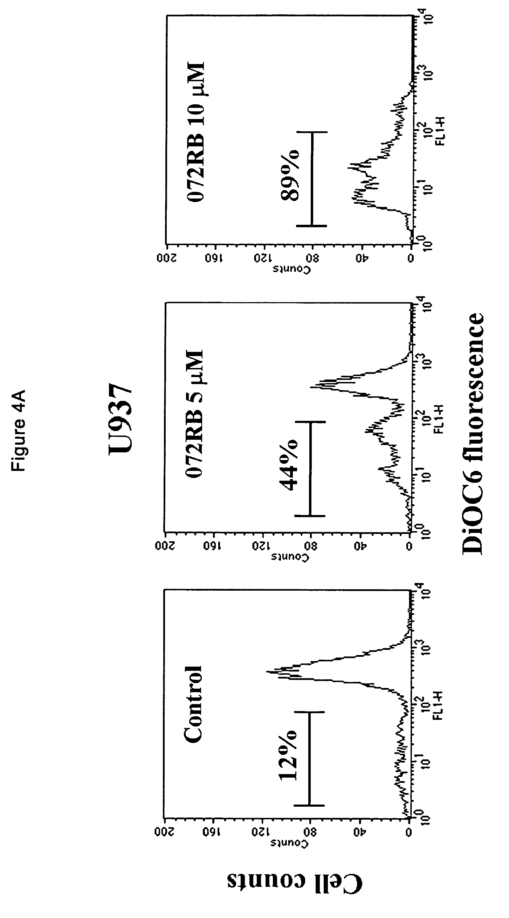

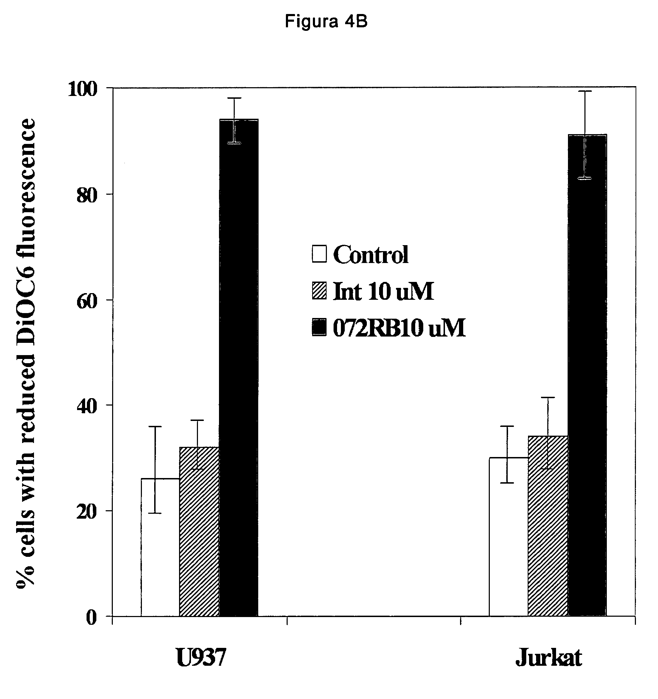

We investigated also whether MPT was involved in 072RB-induced apoptosis. MPT is due to opening of mitochondrial channels and results in a dissipation of the mitochondrial inner membrane potential. MPT was measured as decreased fluorescence of DiOC₆, a lipophylic cationic probe that localizes in the mitochondria of viable cells because of the relatively high electric potential across the mitochondrial inner membrane. DiOC₆ fluorescence histograms obtained in one representative experiment in U937 cells are shown in Figure 4A, and indicate a loss of mitochondrial membrane potential in a proportion of cells at 5 µM and in a larger fraction of them at 10 µM drug concentration. Data obtained by two independent experiments on two cell lines are reported in the bar graph of Figure 4B and confirm clear MPT effects after 072RB treatment.

Following the overall data that clearly demonstrate a significant apoptosis dependent cytotoxic activity of 072RB on human tumour cell lines, we investigated the effects of the peptide on ex-vivo cellular samples from haematological malignancies. Bone marrow aspirates from patients with different leukaemia types were plated in tissue culture medium supplemented with 20% FCS, treated with 072RB twice every 48 h, and followed for 4 days. They were evaluated by flow cytometry DNA/SSC analysis, In Table 3 we show the percentage of [early + late apoptotic cells], according to leukaemia type and Int or 072RB dosage. To further study sensitivity to 072RB of normal cells, peripheral blood lymphocytes (PBL) isolated from four independent healthy donors were subjected to a four-days treatment with 10 µM 072RB. Treatment was performed, as usual, with two pulses at a 48 h interval. Lymphocytes were either un-stimulated or activated by PHA. The effect of 072RB on normal peripheral lymphocytes cell viability and cell proliferation is cytostatic rather than cytotoxic (data not reported). The toxicity of 072RB at 10 µM was quite negligible in quiescent lymphocytes. Comparing treated and untreated normal peripheral lymphocytes, the ratio of viable cells between the two groups was 0.87 ± 0.05 (mean ± SE) for quiescent lymphocytes and 0.82 ± 0.057 for proliferating lymphocytes. This effect of 072RB on normal lymphocytes seems especially small if we consider the effects observed in leukaemia cell lines (see supplementary information), and also the effects observed in AML cells from ex vivo experiments: 0.55 ± 0.145 (mean ± SE).

**Table 3: Effect of 072RB on haematological malignancies (AML: acute myeloid leukaemia; ALL: acute lymphoid leukaemia; NBM: normal bone marrow): percentage of early/late apoptotic cells. Mean values from duplicate experiments are reported. The difference between the two individual values never exceeded 15% of the mean value. Ex vivo isolated cells were treated with either Int or 072RB at day 0, 2 (5, 10 µM), at day 0, 1, 2, 3 (1, 3 µM) and analysed after PI staining at day 4.**

| | | | Int | | 072RB | |
|---|---|---|---|---|---|---|
| Malignancy | FAB Classification | Control | 5 µM | 10 µM | 5 µM | 10 µM |
| AML | M6 | 15 | - | - | - | 67 |
| AML | M2 | 24 | | | 66 | |
| AML | M0 | 33 | 32 | 38 | 36 | 42 |
| AML | M3 | 23 | 26 | 28 | 64 | 82 |
| AML | M0-M1 | 14 | | | 22 | 40 |
| AML | M4-M5 | 50 | | | 75 | |
| ALL | | 43 | | | 67 | |
| ALL | | 40 | | | 42 | 45 |
| ALL | Pro-B | 32 | 32 | 43 | 56 | 63 |
| ALL | | 52 | - | - | 60 | 80 |
| NBM | | 15 | - | 18* | - | 21* |
| NBM | | 39 | - | 37 | - | 39 |

| | | | | | | |
|---|---|---|---|---|---|---|
| * This sample of NBM (normal bone marrow cells) was treated with 15 µM both for Int and 072RB. | | | | | | |

### Activation of caspase 9 and 3 by 072RB

After we demonstrated that 072RB enters the cells and localizes in mitochondria, we further analyzed whether this peptide could trigger mitochondrial apoptosis through the activation of caspase 9 and caspase 3. Cells were incubated for 24, 48 and 72 h with 072RB (10 µM) or Int (10 µM), together with FITC-LEDH-FMK or FITC-DEVD-FMK, to detect caspase 9 and caspase 3 activation, respectively (FACS analysis). After 48 h of incubation a detectable fraction of tumor cells treated with 072RB (30-65% for U937, 15-35% for Namalwa, 10-33% for Jurkat cells, respectively) expressed activated caspase 9, while cells treated with Int alone showed a negligible caspase 9 activation (1.5% for U937, 2.0% for Namalwa, 1.3% for Jurkat cells, respectively). Furthermore, caspase 3, a downstream substrate of caspase 9, was progressively activated at increasing incubation times with 072RB. After 24 h of incubation 30% of U937, 35% of Namalwa, 22% of Jurkat cells, respectively, were positive for activated caspase 3; after 72 h of incubation 70% of U937, 60% of Namalwa, and 35% of Jurkat cells, respectively, had activated caspase 3. On the contrary, a negligible fraction of cells incubated with the Int peptide (1-3%), a % similar to that observed in untreated cells, expressed activated caspase 3.

Finally, we also confirmed that 072RB treated tumor cells undergo apoptosis, in terms of typical alterations of nuclear morphology; by confocal microscopy we analyzed the morphology of PI labeled Namalwa cells after 36 h and 48 h of incubation with 072RB, or after 48 h of incubation with the Int peptide alone; only cells treated with 072RB, but not cells treated with the Int peptide alone, exhibited chromatin condensation and apoptotic body formation.

### References

1. Reed JC. Apoptosis-based therapies. Nat. Rev. Drug Discov. 2002; 1:111-121.
2. Johnstone, R. W., Ruefli, A. A. & Lowe, S. W. Apoptosis: a link between cancer genetics and chemotherapy. Cell 2002, 108: 153-164.
3. Adams J, and Cory S. The Bcl-2 protein family: arbiters of cell survival. Science 1998, 281: 1322-1326.
4. Cory S., Adams J. The Bcl-2 family: regulators of the cellular life or death switch. Nat.Rew. Cancer 2002, 2: 647-656.
5. Kroemer G, Reed JC. Mitochondrial control of cell death. Nat Med. 2000, 6: 513-519.
6. Cory S, Huang D. CS and Adams J. M. The Bcl-2 family: roles in cell survival and oncogenesis. Oncogene 2003, 22; 8590-8607.
7. Holinger E.P., Chittenden T., and Lutz R. Bak BH3 peptides antagonize Bcl-xL function and induce apoptosis through cytochrome c-independent activation of caspases. J Biol. Chem 1999, 274: 13298-13304.
8. Moreau C., Cartron PF., Hunt A., Meflah K., Green D.R., Evan G., et al.Minimal BH3 peptides promote cell death by antagonizing anti-apoptotic proteins. JBC 2003, 278: 19426-19435.
9. Minn, A.J., Rudin, C. M., Boise, L.H., and Thompson, C.B. Expression of Bcl-XL can confer a multidrug resistance phenotype. Blood 1995, 86: 1903-1910.
10. Amundson S. A., Myers T.y G., Scudiero D., Kitada S., Reed J.C., and Fornace A.J. Jr, An Informatics Approach Identifying Markers of Chemosensitivity in Human Cancer Cell Lines. Cancer Research 2000, 60: 6101-6110..
11. Walensky L.D.,. Kung A. L, Escher I., Malia T.J., Barbuto S., Wright R.D., et al, Activation of Apoptosis in Vivo by a Hydrocarbon-Stapled BH3 Helix. Science 2004, 305: 1466-1470.
12. Oltersdorf T, Elmore SW, Shoemaker AR, Armstrong RC, Augeri DJ, Belli BA, et al. An inhibitor of Bcl-2 family proteins induces regression of solid tumours. Nature. 2005, 2: 677-681.
13. Letai A., Bassik M.C., Wlenky L.D., Sarcinelli M.D., Weiler S., Korsmeyer S,J, Distinct BH3 domains either sensitize or activate mitochondrial apoptosis, serving a prototype cancer therapeutics. Cancer Cell 2002, 3: 183-192.
14. Wang, J.L., Liu, D., Zhang, Z.J., Shan, S., Han, X., Srinivasula, S.M., et al. Structure-based discovery of an organic compound that binds Bcl-2 protein and induces apoptosis of tumor cells. Proc. Natl. Acad. Sci. 2000, 97; 7124-7129.
15. Wu S., Zhu H., Gu J., Zhang L., Teraishi F., Davis J.J. et al. Induction of Apoptosis and Down-ltegulation of Bcl-XL in Cancer Cells by a Novel Small Molecule, 2[[3-(2,3-Dichlorophenoxy)propyl]amino]ethanor. Cancer Research 2004, 64: 1110-1113.
16. Letai A. BH3 domains as BCL-2 inhibitors; prototype cancer therapeutics, Expert Opin Biol Ther. 2003, 3(2): 293-304.
17. O'Connor L., Strasser A., O'Reilly L.A., Husmann G., Adams J.M., Cory S., et al. Bim: a novel member of the Bcl-2 family that promotes apoptosis. The Embo journal 1998, 17: 384-395.
18. Liu X., Dai S., Zhu Y., Marrack P., Kappler J.W. The structure of Bcl-XI Bim fragment complex: implication for Bim function. Immunity 2003, 19: 341-352.
19. Derossi, D., Calvet, S., Trembleau, A., Brunissen, A., Chassaing, G., Prochiantz, A. Cell internalization of the third helix of the Antennapedia Homeodomain is receptor-independent. J. Biol. Chem. 1996, 271: 18188-18193.
20. Wellings, D.; Atherton, E.; Standard Fmoc Protocols. Methods Enzimol. 1997, 4, 289.
21. Thompson, J.D., Higgins, D.G. and Gibson, T.J. Nucleic Acids Res 1994, 22: 4673-80.
22. Nicholas, K.B., Nicholas H.B. Jr., and Deerfield, D.W. II. EMBNEW.NEWS, 1997, 4: 14.
23. Lundblad J.R., Laurance M., Goodman R.H. Fluorescence Polarization Analysis of protein-dna and protein-protein interactions. Molecular Endocrinology 1996, 10: 607-612.
24. Davison, A. C. & D. V. Hinkley. 1997. Bootstrap Methods and their Application. Cambridge: Cambridge University Press.
25. Zhang H., Nimmer P., Rosenberg S. H., Ng S. C., Joseph M., Development of high-throughput fluorescence polarization assay for Bel-XL. Analytical Biochemistry 2001, 207: 70-75.
26. Qian J., Voorbach M.J., Hunth J. R., Coen M.L., Zhang H., Ng S.C et al. Discovery of novel inhibitors of Bcl XL using multiple high-throughput screening platforms. Analytical Biochemistry 2004, 328: 131-138.
27. Sattler, M., Liang, H., Nettesheim, D., Meadows, R.P., Harlan, J.E., Eberstadt, M. et al. Structure of Bcl-xL-Bak peptide complex: recognition between regulators of apoptosis. Science 1997, 275: 983-986.
28. Karle, I.L.; Baslaram, P. Structural characteristics of .alpha.-helical peptide molecules containing Aib residues. Biochemistry 1990, 29; 6747-6756.
29. Andrews M. J. I., Tabor A. B. Forming stable helical peptides using natural and artificial amino acids. Tetrahedron 1999, 55: 11711-11743.
30. Alain Prochiantz. Getting hydrophilic compounds into cells: lessons from homeopeptides. Current Opinion in Neurobiology 1996, 6: 629-634.
31. Petros, A.M.; Nettesheim, D.G,; Wang, Y.; Olejniczak, E.T.; Meadows, R.P.; Mack, J. et al. Rationale for Bcl-xL/Bad peptide complex formation from structure, mutagenesis, and biophysical studies Protein Sci 2000, 9: 2528-2534.
32. Johnson, W.C. Analyzing protein circular dichroism spectra for accurate secondary structures. Proteins 1999, 35: 307-312. Thompson J.D., Higgins D.G. and Gibson T.J, Nucleic Acids Res. 1994; 22: 4673-4680.
33. Vijayakumar, E. K. S.; Sudha, T. S.; Balaram, P. Circular dichroism studies of -aminoisobutyric acid-containing peptides: Chain length and solvent effects in alternating Aib-L-Ala and Aib-L-Val sequences, Biopolymers 1984, 23: 877-886. Nicholas, K.B., Nicholas H.B. Jr., and Deerfield, D.W. II. EMBNEW.NEWS. 1997; 4: 14.
34. Del Principe MI, Del Poeta G, Venditti A, Buccisano F, Maurillo L, Mazzone C, et al. Apoptosis and immaturity in acute myeloid leukemia. Hematology. 2005, 10: 25-34.
35. Licht JD, Sternberg DW. The molecular pathology of acute myeloid leukemia. Hematology Am Soc Hematol Educ Program. 2005; 137-42.
36. Goldsmith KC, Liu X, Dam V, Morgan BT, Shabbout M, Cnaan A et al. BH3 peptidomimetics potently activate apoptosis and demonstrate single agent efficacy in neuroblastoma. Oncogene. 2006, 3: 4525-33.
37. Ooi SL, Pan X, Peyser BD, Ye P, Meluh PB, Yuan DS et al. Global synthetic-lethality analysis and yeast functional profiling. Trends Genet. 2006, 22: 56-63.

## Claims

1. A pro-apoptotic peptide having the amino acid sequence (I):
Bₙ**X**₁ **X**₂ **X**₃ **X**₄ **X**₅ IYIAib**X**₆ **X**₇L**X**₈ **X**₉IGDAF**X**₁₀Aib**X**₁₁Bₘ (I)
wherein:
B represents a cellular internalization sequence which is selected from:
KKWKMRRNQFWIKIQR
KKWKMRRNPFWIKIQR
GRKKRRQRRRPPQ
RKKRROrnRRRPPQ
(A,xg)n, wherein n ranges from 4 to 16;
PheLeuPheLeu,
or corresponding retro-inverted sequences,
n and m are 0 or 1, provided they are not both 1;
**X**₁ is selected from aspartic acid, glutamic acid, asparagine, glycine, alanine, serine;
**X**₂ is selected from methionine, leucine, lysine, valine, isoleucine, alanine;
**X**₃ is selected from arginine, lysine, serine, glutamine, histidine, proline;
**X**₄ is selected from proline, alanine, serine, glutamine, threonine, valine, histidine;
**X**₅ is selected from glutamic acid, aspartic acid, glutamine, alanine, glycine, lysine;
**X**₆ is selected from glutamine, glutamic acid, histidine, lysine, arginine, alanine, proline;
**X**₇ is selected from glutamic acid, aspartic acid, glutamine, alanine, glycine, lysine;
**X**₈ is selected from arginine, lysine, serine, glutamine, histidine, proline;
**X**₉ is selected from arginine, lysine, serine, glutamine, histidine, proline;
**X**₁₀ is selected from asparagine, aspartic acid, serine, lysine, histidine, threonine, glycine;
**X**₁₁ is selected from tyrosine, phenylalanine, histidine, asparagine, cysteine, leucine, threonine;
Aib is alpha-amino isobutyric acid;
and wherein the indicated amino acid residues are in either L or D configuration.

2. A peptide according to claim 1, wherein the residues at C- or N-terminus of the cellular internalization sequence B are linked to C10 or C14 fatty acids by esteric or amidic bonds.

3. A peptide according to claim 2, wherein said cellular internalization sequence B is KKWKMRRNPFWIKIQR.

4. A peptide according to claims 1-3, wherein m is 1 and n is 0.

5. A peptide according to claims 1-4, wherein:
**X**₁ is selected from aspartic acid, glutamic acid and asparagine;
**X**₂ is selected from methionine, leucine and lysine;
**X**₃ is selected from arginine, lysine and serine;
**X**₄ is selected from proline, alanine and serine;
**X**₅ is selected from glutamic acid, aspartic acid and glutamine;
**X**₆ is selected from glutamine, glutamic acid and histidine;
**X**₇ is selected from glutamic acid, aspartic acid and glutamine;
**X**₈ is selected from arginine, lysine and serine;
**X**₉ is selected from arginine, lysine and serine;
**X**₁₀ is selected from asparagine, aspartic acid and serine;
**X**₁₁ is selected from tyrosine, phenylalanine and histidine;

6. A peptide according to claim 5, wherein:
**X**₁ is aspartic acid;
**X**₂ is methionine;
**X**₃ is arginine;
**X**₄ is proline;
**X**₅ is glutamic acid;
**X**₆ is glutamine;
**X**₇ is glutamic acid;
**X**₈ is arginine;
**X**₉ is arginine;
**X**₁₀ is asparagine;
**X**₁₁ is tyrosine;

7. A peptide according to claims 1-6, carrying allylic groups attached to amidic nitrogens or to the hydroxyl functionalities present in Ser, Tyr or Gly residues.

8. A peptide according to claim 1, which is:
BₙDMRPEIYIAibQELRR:1GDAFNAibYBₘ wherein Aib, B, n and m are as above defined.

9. A peptide according to claim 8, which is:
DMRPEIYIAibQELRRIGDAFNAibYKKWKMRRNPFWIKIQR.

10. A pharmaceutical composition containing a peptide according to claims 1-9 in combination with pharmaceutically acceptable carriers or excipients.

11. The use of a peptide according to claims 1-9 for the preparation of a medicament for the prevention or treatment of proliferative diseases that involve a defective or uncotrolled apoptotic process.

12. The use according to claim 11, wherein said diseases is selected from cancer, polycitemia, thrombocythemia, idiopathic myelofibrosis.

13. The use according to claim 12, wherein said cancer is selected from leukemia, lymphoma, carcinoma, glioma and sarcoma.

14. The use according to claim 13, wherein said cancer is acute myeloid leukemia or acute lymphoblastic leukemia.
